(19) Europäisches Patentamt / European Patent Office / Office européen des brevets

(11) **EP 4 506 956 A1**

(12) **EUROPEAN PATENT APPLICATION**
published in accordance with Art. 153(4) EPC

(43) Date of publication:
**12.02.2025 Bulletin 2025/07**

(21) Application number: **22941410.7**

(22) Date of filing: **31.08.2022**

(51) International Patent Classification (IPC):
**G16H 50/20** (2018.01)

(86) International application number:
**PCT/CN2022/116248**

(87) International publication number:
**WO 2023/216469 (16.11.2023 Gazette 2023/46)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **09.05.2022 CN 202210496595**

(71) Applicant: **Shenzhen Bay Laboratory
Shenzhen, Guangdong 518132 (CN)**

(72) Inventor: **SUN, Kun
Shenzhen, Guangdong 518132 (CN)**

(74) Representative: **Nex & Phister Law & IP Aps
Symbion Fuglebakken
Nordre Fasanvej 215
2000 Fredriksberg (DK)**

(54) **CELL-FREE DNA-BASED CANCER DIAGNOSIS MODEL AND USE**

(57) Disclosed in the present invention are a cell-free DNA-based cancer diagnosis model and the use. In a first aspect of the present application, provided is a construction method of a cancer diagnosis model, the construction method comprising the following steps: acquiring sequencing data of cell-free DNAs of a non-cancer population; comparing the sequencing data of the cell-free DNAs with a reference genome to obtain corresponding sites of the tail ends of the cell-free DNAs on the reference genome; and constructing a cancer diagnosis model according to the corresponding sites of the tail ends of the cell-free DNAs on a target genome range. The present application uses an idea opposite to a mainstream method, and constructs a cancer diagnosis model by means of searching for a tail end distribution mode of cfDNAs of a non-cancer population, instead of attempting to identify a diagnosis marker based on a tumor cfDNA specific fragmentation mode. By means of the manner, patients with a plurality types of different cancers can be distinguished from a contrast population, achieving good specificity and sensitivity, and realizing pancancer type cancer diagnosis.

```
┌─────────────────────────────────────────────────────────────┐
│   obtaining end distribution data of cell-free DNA of samples │  ~S210
└─────────────────────────────────────────────────────────────┘
                              │
                              ▼
┌─────────────────────────────────────────────────────────────┐
│  comparing the end distribution data of the cell-free DNA of  │  ~S220
│  the samples with the end distribution data of the cell-free  │
│  DNA of the non-cancer population                             │
└─────────────────────────────────────────────────────────────┘
                              │
                              ▼
┌─────────────────────────────────────────────────────────────┐
│  determining the classification of the samples based on       │  ~S230
│  comparison results                                           │
└─────────────────────────────────────────────────────────────┘
```

FIG.2

## Description

### TECHNICAL FIELD

The present disclosure relates to the technical field of cancer diagnoses, and in particular, to a cell-free DNA-based cancer diagnosis model and use.

### BACKGROUND

Circulating cell-free DNA (hereinafter referred to as the "cfDNA") in peripheral blood is a DNA fragment generated by natural degradation after cell death. Studies show that the main sources of cfDNA are blood cells and liver in healthy people. However, in cancer patients, dead tumor cells also release cfDNA into the peripheral blood. There are many differences between cfDNA fragments from tumors and cfDNA fragments from non-tumor cells, such as gene mutations, chromosome copy number variations, abnormal methylation levels, and the like. Therefore, cancer diagnoses may be carried out by detecting cfDNA signals from tumors in peripheral blood. That is, liquid biopsy technology. Since the collection of peripheral blood is almost non-invasive to the human body and can be sampled repeatedly, the cancer diagnosis technology based on peripheral blood cfDNA is of high application value. According to related studies, a large number of tumor cfDNA markers have also been identified, and a large number of cancer diagnostic technologies have been developed, such as high-frequency gene mutation kits, tumor-specific methylation marker kits, and the like.

In recent years, researchers have discovered that cfDNA is not randomly fragmented when generated. Based on this principle, they have discovered some tumor markers associated with cfDNA fragmentation patterns and developed multiple cancer diagnostic technologies. For example, liver cancer patients have a set of preferential cfDNA end sites distributed on a genome. These preferential end sites can be used to distinguish liver cancer patients from the control group and can therefore be used as tumor markers. However, sophisticated statistical methods are required to identify these preferential sites. Moreover, each cancer needs to be identified individually, which is costly and has poor universality. In addition, the diagnosis based on cfDNA length and end base usage pattern is of much poor accuracy, and the sensitivity is very limited in early-stage cancer patients. Therefore, it is necessary to provide a cfDNA-based cancer diagnosis model with strong universality and high accuracy.

### SUMMARY

The present disclosure aims to solve at least one of the technical problems existing in the prior art. To this end, a cfDNA-based cancer diagnosis model with strong universality and high accuracy and use are proposed herein.

According to a first aspect of the present disclosure, a construction method for a cancer diagnosis model is provided, including the following steps:

obtaining sequencing data of cell-free DNA of a non-cancer population;

comparing the sequencing data of the cell-free DNA with reference genomes to obtain corresponding sites of the ends of the cell-free DNA on the reference genomes; and

constructing the cancer diagnosis model based on the corresponding sites of the ends of the cell-free DNA on target genome intervals.

The construction method according to an embodiment of the present disclosure has at least the following beneficial effects:

In the peripheral blood of the non-cancer population, cfDNA usually comes from normal cells, and the overall fragmentation pattern is relatively similar and is of high consistency. In cancer patients, due to the high heterogeneity of the tumors themselves, the fragmentation patterns of cfDNA released by tumors of different cancer types, or even different patients with the same cancer type, may be very different, thereby resulting in tumor markers identified based on tumor patient samples being generally low in universality and sensitivity. From another perspective, although the cfDNA fragmentation patterns of each tumor patient may be different from each other, they all have one thing in common. That is, they are different from the DNA fragmentation patterns of the non-cancer population. Based on this principle, an idea that is opposite to the prevailing method is adopted herein. Instead of trying to identify diagnostic markers based on the specific fragmentation patterns of tumor cfDNA, the applicant constructs the cancer diagnosis model by looking for the end distribution patterns of cfDNA in the non-cancer population. In this way, patients of a variety of different cancers may be distinguished from the control population, with good specificity and sensitivity, thus achieving pan-cancer cancer diagnosis.

In some embodiments of the present disclosure, the ends of the cell-free DNA include at least one of upstream ends and downstream ends.

In some embodiments of the present disclosure, a reference genome is a whole human genome or a set interval of the whole human genome or may be a genome of someone or multiple human individuals.

In some embodiments of the present disclosure, a target genome interval is the whole human genome or the set interval of the whole human genome.

In some embodiments of the present disclosure, the target genome interval is at least one of chr1, chr2, chr3, chr4, chr5, chr6, chr7, chr8, chr9, chr10, chr11, chr12, chr13, chr14, chr15, chr16, chr17, chr18, chr19, chr20, chr21, chr22, chrX, and chrY in the whole human genome.

The reference genome and the target genome interval may be the same or different, but they obviously have overlapping portions.

In some embodiments of the present disclosure, a construction method for the cancer diagnosis model based on the corresponding sites of the ends of the cell-free DNA on the target genome intervals comprises:

calculating the number of occurrences that different sites of the target genome intervals appear as the ends of the cell-free DNA to construct an end distribution model that represents a site-frequency relationship, namely being the cancer diagnosis model.

According to a second aspect of the present disclosure, a sample classification method is provided, including the following steps:

obtaining end distribution data of cell-free DNA of samples;

comparing the end distribution data of the cell-free DNA of the samples with the end distribution data of the cell-free DNA of a non-cancer population; and

determining the classification of the samples based on comparison results.

In some embodiments of the present disclosure, the method for obtaining the end distribution data of the cell-free DNA of the samples includes:

obtaining sequencing data of the cell-free DNA of the samples;

comparing the sequencing data of the cell-free DNA of the samples with reference genomes to obtain corresponding sites of the ends of the cell-free DNA of the samples on the reference genomes; and

calculating the number of occurrences that different sites of target genome intervals appear as the ends of the cell-free DNA of the samples.

In some embodiments of the present disclosure, the end distribution data of the cell-free DNA of the non-cancer population is obtained by:

obtaining sequencing data of the cell-free DNA of the non-cancer population;

comparing the sequencing data of the cell-free DNA of the non-cancer population with the reference genomes to obtain the corresponding sites of the ends of the cell-free DNA of the non-cancer population on the reference genomes; and

calculating the number of occurrences that different sites of the target genome intervals appear as the ends of the cell-free DNA of the non-cancer population to construct an end distribution model that represents a site-frequency relationship.

In some embodiments of the present disclosure, the method of comparison includes comparing a consistency of the number of occurrences that different sites of the target genome intervals appear as the ends of the cell-free DNA of the samples and the number of occurrences that different sites of the target genome intervals appear as the ends of the cell-free DNA of the non-cancer population;

the consistency is compared by comparing the correlation of both the number of occurrences that different sites of the target genome intervals appear as the ends of the cell-free DNA of the samples and the number of occurrences that different sites of the target genome intervals appear as the ends of the cell-free DNA of the non-cancer population or calculating an end distribution consistency by the following formula: $\mathrm{E-index} = \sum_{i=1}^{n} a_i b_i$ ; and where $n$ is the number of sites appearing in the end distribution model, $a_i$ is the frequency of site $i$ appearing in the end distribution model, and $b_i$ is the frequency of site $i$ appearing in the ends of the cell-free DNA of the samples.

In some embodiments of the present disclosure, the method for determining the classification of the samples based on the comparison results and the end distribution consistency includes:
comparing the end distribution consistency (E-index) of the samples with a cut off value, where when the end distribution consistency is greater than or equal to the cut off value, the samples are classified as non-cancer samples; and when the end distribution consistency is less than the cut off value, the samples are classified as cancer samples.

In some embodiments of the present disclosure, the cancer is selected from at least one of brain cancer, head and neck cancer, lung cancer, liver cancer, breast cancer, stomach cancer, colon cancer, rectal cancer, cervical cancer, ovarian cancer, pancreatic cancer, prostate cancer, thyroid cancer, lymphoma, skin cancer, and leukemia.

In some embodiments of the present disclosure, the classification method further includes obtaining at least one of copy number variation data of cell-free DNA, methylation data of cell-free DNA, length distribution data of cell-free DNA, and end base distribution data of cell-free DNA, marker data, and image data of the samples for analysis, and determining the classification of the samples by combining analysis results with the comparison results.

According to a third aspect of the present disclosure, a cancer diagnosis model that is constructed by using the aforementioned method is provided.

According to a fourth aspect of the present disclosure, a computer-readable storage medium storing computer-executable instructions is provided, where the computer-executable instructions are used to cause the computer to execute the aforementioned construction method or the aforementioned classification method.

According to a fifth aspect of the present disclosure, a device including a processor and a memory is provided, where the memory stores a computer program capable of running on the processor, and the processor implements the aforementioned construction method or the aforementioned classification method when running the computer program.

According to a sixth aspect of the present disclosure, a device for constructing a cancer diagnosis model is provided, including:

an acquisition module configured to obtain sequencing data of cell-free DNA of a non-cancer population;

a comparison module configured to compare the sequencing data of the cell-free DNA of the non-cancer population with reference genomes to obtain corresponding sites of the ends of the cell-free DNA on the reference genomes; and

a model construction module configured to construct the cancer diagnosis model based on the corresponding sites of the ends of the cell-free DNA on target genome intervals.

According to a seventh aspect of the present disclosure, a device for diagnosing cancer is provided, including:

an acquisition module configured to obtain end distribution data of cell-free DNA of samples; and

a comparison module configured to compare the end distribution data of the cell-free DNA of the samples with the end distribution data of the cell-free DNA of the non-cancer population and determine whether the samples are cancer samples according to the comparison results.

Based on the above, it can be seen that a new cancer diagnosis-related method based on the end distribution patterns of peripheral blood cfDNA is proposed herein. The experiments required to obtain cfDNA data may use commonly used whole genome shotgun sequencing, with simple experimental operations and without high initial materials requirements. However, the diagnosis model may utilize most of the data information, with low diagnosis costs and without high sequencing depth requirements. In addition, this technology has the following characteristics of:

1) not using cancer patient samples to identify tumor markers;

2) not using statistical methods to identify tumor markers;

3) using the diagnosis model in a plurality of cancer types (i.e., the cancer diagnosis of pan-cancer may be achieved); and

4) no specific requirements for the chemical reagents and sequencing platforms used in the experiments by the diagnosis model (high universality).

Additional aspects and advantages of the present disclosure will be set forth in part in the description which follows, and in part will be obvious from the description, or may be learned by practices of the present disclosure.

**BRIEF DESCRIPTION OF DRAWINGS**

FIG. 1 is a schematic flowchart of a method for constructing a cancer diagnosis model provided by an embodiment of the present disclosure.

FIG. 2 is a schematic flowchart of a sample classification method provided by an embodiment of the present disclosure.

FIG. 3 is a schematic diagram of the whole process of cancer diagnosis in Example 1 of the present disclosure.

FIG. 4 is a comparison result of an E-index calculated for the samples of data set 1 in Example 1 of the present disclosure.

FIG. 5 is an ROC curve of the E-index of liver cancer/lung cancer and the control group in data set 1 in Example 1 of the present disclosure.

FIG. 6 is a correlation test result of the E-index and tumor DNA abundance of cancer patients in data set 1 in Example 1 of the present disclosure.

FIG. 7 is a comparison result of the E-index calculated for the samples of data set 2 in Example 1 of the present disclosure.

FIG. 8 is an ROC curve of the E-index of different cancers and the control group in data set 2 in Example 1 of the present disclosure.

FIG. 9 is a comparison result of the E-index of lung cancer with metastasis and non-metastasis in data set 2 in Example 1 of the present disclosure.

FIG. 10 is an ROC curve of the E-index of lung cancer metastasis and non-metastasis in data set 2 in Example 1 of the present disclosure.

FIG. 11 is a comparison result of the E-index calculated for the samples of data set 3 in Example 1 of the present disclosure.

FIG. 12 is an ROC curve of the E-index of different cancers and the control group in data set 3 in Example 1 of the present disclosure.

**DETAILED DESCRIPTION**

The concept of the present disclosure and the technical effects produced will be clearly and completely described below in conjunction with the embodiments to fully understand the objectives, features and effects of the present disclosure. Obviously, the described embodiments are only some of the embodiments of the present disclosure, rather than all of the embodiments. Based on the embodiments of the present disclosure, other embodiments obtained by those skilled in the art without exerting creative efforts are all fall within the scope of protection of the present disclosure.

The embodiments of the present disclosure are described in detail below. The described embodiments are exemplary and only used for explaining the present disclosure, and cannot be understood as limiting the present disclosure.

In the description of the embodiments of the present disclosure, the meaning of "a plurality of " refers to one or more, and the meaning of "multiple" refers to two or more. The terms such as "greater than", "less than", "over" are understood not to include the specified number, while the terms such as "above", "below", "within" are understood to include the specified number. If described, the terms such as "first", "second" are merely for the purpose of distinguishing technical features, and not to be construed as indicating or implying relative importance or implicitly indicating the number of technical features indicated or implicitly indicating the precedence relationship of technical features indicated. If a logical sequence is depicted in the flowcharts, under certain circumstances, the steps described or illustrated may be performed in a sequence different from that in the flowcharts.

In the description of the present disclosure, reference to the terms "one embodiment", "some embodiments", "illustrative embodiments", "examples", "specific examples" or "some examples" means that the specific features, structures, materials, or characteristics described in conjunction with the embodiment or example are included in at least one embodiment or example of the present disclosure. In this specification, schematic representations of the above terms do not necessarily refer to the same embodiment or example. Furthermore, the specific features, structures, materials or characteristics described may be combined in any suitable manner in any one or more embodiments or examples.

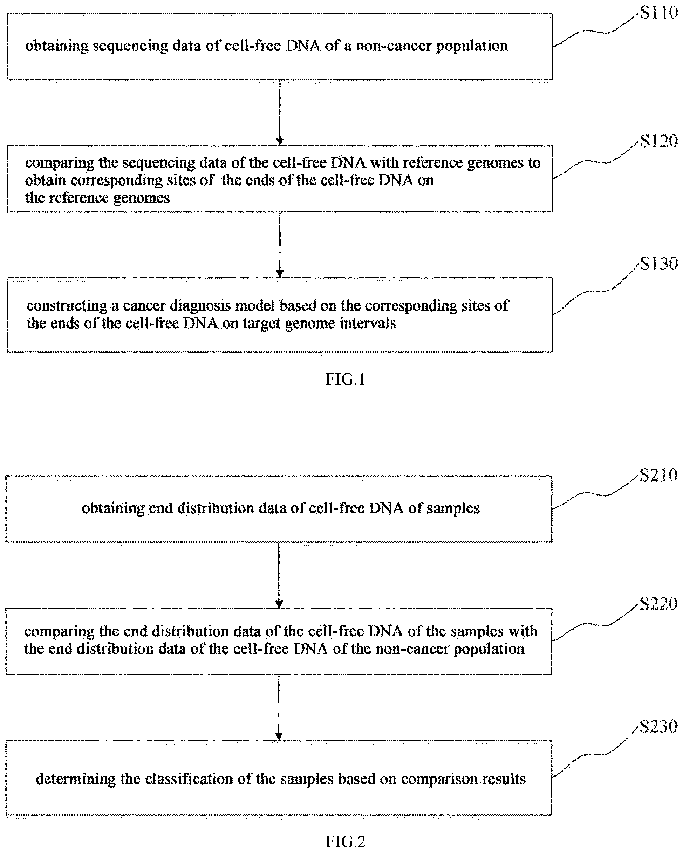

The present disclosure provides a construction method for a cancer diagnosis model. Referring to FIG. 1, the method includes the following steps:

S110: Sequencing data of cell-free DNA of a non-cancer population is obtained. Specifically, the non-cancer population refers to a collection of people who do not suffer from cancer. For example, they may be those people who have been confirmed not to have cancer through one or more methods such as tissue, cells, molecules, immunity, imaging, and the like. Specifically, the non-cancer population may be healthy people or people suffering from other diseases besides cancer (such as underlying diseases, including high blood pressure, diabetes, etc.). There is no limit to the number of non-cancer population. For example, the number of non-cancer population may be 1 to 10,000 or more, and further it may be more than 5, more than 10, more than 20, more than 30, more than 50, or more than 100. Specifically, it may be 1, 2, 5, 10, 20, 50, 100, 200, 500, 1,000, 2,000, 5,000, 10,000. Cell-free DNA (or cfDNA) refer to plasma cell-free DNA, which usually refer to extracellular nucleic acid fragments in peripheral blood circulation.

S120: The sequencing data of the cell-free DNA is compared with reference genomes to obtain corresponding sites of the ends of the cell-free DNA on the reference genomes. In this step, by comparing the sequencing data of the cell-free DNA with the reference genomes, the cell-free DNA are positioned on the reference genomes to obtain the positioning sites of the ends of the cell-free DNA on the reference genomes. Specifically, the reference genome is an optional human reference genome, including but not limited to any one of GRCh36 (hg18), GRCh37 (hg19), GRCh38 (hg38), etc. Of course, the reference genome is not limited to this. It may also be a specific interval on the human reference genome, and only some of the intervals of interest are compared during the comparison. In addition, the genome of one or more human individuals or a specific interval thereon may also be considered. It can be understood that, depending on the different sequencing methods, the ends of the cell-free DNA may refer to one end or both ends of the cell-free DNA. Specifically, when using single-end sequencing, only the positioning site of a single end on the reference genome is selected in this step. In paired-end sequencing, the ends of the cell-free DNA may choose either the upstream end, the downstream end, or all positioning sites of both ends on the reference genomes to participate in the construction of the model. The upstream end and the downstream end refer to the upstream or downstream of cfDNA sequencing. The end and positioned site usually refer to the end base and the corresponding site of the base on the reference genome.

S130: The cancer diagnosis model is constructed based on the corresponding sites of the ends of the cell-free DNA on target genome intervals. Specifically, the cancer diagnosis model includes an end distribution model of the cell-free DNA in the non-cancer population. The end distribution model of the cell-free DNA in the non-cancer population refers to a model that represents the end distribution pattern of the cell-free DNA in the non-cancer population. The end distribution pattern refers to the distribution characteristics of the ends of the cell-free DNA in the non-cancer population at different sites in the target genome intervals. In some specific embodiments, this distribution characteristic is reflected by the frequency of occurrence of the ends of the cell-free DNA at different sites on the target genome intervals. To this end, based on the corresponding sites of the ends of the cell-free DNA on the target genome intervals, the method for constructing the end distribution model of cell-free DNA in the non-cancer population includes: calculating the number of occurrences that different sites of the target genome intervals appear as the ends of the cell-free DNA to construct an end distribution model that represents a site-frequency relationship, and using this end distribution model of cfDNA in the non-cancer population as a cancer diagnosis model. In addition, the target genome interval may be the whole human genome, or a set interval in the whole human genome, such as some chromosomes, such as any one or more of chr1~22, chrX, and chrY, or one or more intervals customized in the whole human genome. It should be noted that if a set interval in the whole human genome is used, it may be achieved by at least one or more of the following methods, such as using capture or enrichment technology (such as target capture, whole exome sequencing, and the like) during sequencing before obtaining data, or only selecting DNA sequencing reads in the set interval when obtaining data. The reference genome and the target genome interval may be the same or different, but obviously, they need to have overlapping parts to complete the construction of the model, and in some cases, the reference genome covers the target genome interval.

In the peripheral blood of the non-cancer population, cfDNA usually come from normal cells, and the overall fragmentation pattern is relatively similar and is of high consistency. In cancer patients, due to the high heterogeneity of the tumors themselves, the fragmentation patterns of cfDNA released by tumors of different cancer types, or even different patients of the same cancer type, may be very different, thereby resulting in tumor markers identified based on tumor patient samples being generally low in universality and sensitivity. From another perspective, although the cfDNA fragmentation patterns of each tumor patient may be different from each other, they all have one thing in common, that is, they are different from the DNA fragmentation patterns of the non-cancer population. Based on this principle, an idea that is opposite to the prevailing method is adopted herein. Instead of trying to identify diagnostic markers based on the specific fragmentation patterns of tumor cfDNA, the applicant constructs the cancer diagnosis model by looking for the end distribution patterns of cfDNA in the non-cancer population. In this way, patients of a variety of different cancers may be distinguished from the control population, with good specificity and sensitivity, thus achieving the cancer diagnosis of pan-cancer.

The present disclosure further provides a cancer diagnosis model constructed according to the above methods. Different from common tumor markers, this model describes the end distribution law of cfDNA released by non-tumor cells (or "normal" or "background") in peripheral blood. These non-tumor-derived cfDNA fragmentation patterns are relatively similar. Thus, the consistency between models constructed using samples from different types of the non-cancer population is high, and is not affected by tumor heterogeneity. Compared with the identification of tumor-specific markers, this model has higher universality and provides strong support for the pan-cancer diagnosis technology.

Additionally, the model eliminates the need for statistical analysis to screen biomarkers, instead of incorporating all data into the model. This is because when the amount of data is large enough, the biological laws behind have already made it better conform to the specific distribution law, and there is no need for statistical analysis to screen. This method may reduce the complexity of constructing models and avoid various errors caused by improper use of statistical models. It may also improve data utilization and reduce sequencing depth requirements.

The present disclosure further provides a sample classification method. Referring to FIG. 2, the classification method includes the following steps:

S210: The end distribution data of the cell-free DNA of the samples is obtained. The end distribution data of the cell-free DNA refers to the positioning of the ends of the cell-free DNA in the target genome intervals. Specifically, it refers to the distribution pattern of one or two ends of a plurality of cell-free DNA covering all or part of a sample, described in mathematical language, on the positioning sites in the target genomic intervals. To this end, the method for obtaining end distribution data of the cell-free DNA of the samples includes: obtaining sequencing data of the cell-free DNA of the samples; comparing the sequencing data of the cell-free DNA of the samples with reference genomes to obtain the corresponding sites of the ends of each cell-free DNA of the samples on the reference genomes; and counting the corresponding sites of the ends of different cell-free DNA on the target genome intervals to calculate the number of occurrences that different sites of the target genome intervals appear as the ends of the cell-free DNA of the samples. In this step, the reference genome or target genome interval may be an optional human reference genome, including but not limited to any one of GRCh36 (hg18), GRCh37 (hg19), GRCh38 (hg38), etc. Of course, both the reference genome or target genome interval are not limited to this. They may also be a specific interval on the human reference genome or the genome of a certain human individual or a specific interval thereon. The specific examples include but are not limited to some chromosomes, such as any one or more of chr1~22, chrX, and chrY, or one or more intervals customized in the whole human genome. The reference genome and the target genome interval may be the same or different, but obviously, they need to have overlapping portions, and in some cases, the reference genome covers the target genome interval. At the same time, depending on the different sequencing methods, the ends of the cell-free DNA may refer to one end or both ends of the cell-free DNA.

S220: The end distribution data of the cell-free DNA of the samples is compared with the end distribution data of the cell-free DNA of the non-cancer population. In this step, the end distribution data of the cell-free DNA of the non-cancer population is obtained by: obtaining sequencing data of the cell-free DNA of the non-cancer population; comparing the sequencing data of the cell-free DNA of the non-cancer population with the reference genomes to obtain the corresponding sites of the ends of the cell-free DNA of the non-cancer population on the reference genomes; and calculating the number of occurrences that different sites of the target genome intervals appear as the ends of the cell-free DNA of the non-cancer population and obtaining a statistical table of the number of occurrences that each site appears as the end of cell-free DNA to construct an end distribution model that represents a site-frequency relationship. For details, reference may be made to the first aspect of an embodiment of the present disclosure.

In some of the specific embodiments, the method for comparing the end distribution data of the cell-free DNA of the samples and the end distribution data of the cell-free DNA of the non-cancer population includes comparing a consistency of the number of occurrences that different sites of target genome intervals appear as the ends of the cell-free DNA of the samples and the number of occurrences that different sites of the target genome intervals appear as the ends of the cell-free DNA of the non-cancer population. Specifically, the consistency is compared by calculating an end distribution consistency by the following formula: $\text{E} - \text{index} = \sum_{i=1}^{n} a_i b_i$, where $n$ is the number of sites appearing in the end distribution model, $a_i$ is the frequency of site $i$ appearing in the end distribution model, and $bi$ is the frequency of site $i$ appearing in the ends of the cell-free DNA of the samples. In this implementation, the positioning sites of the cell-free DNA and the frequency of occurrence as ends are directly used as the end distribution model, and the model of the non-cancer population is used as the weighting weight to weight the frequency of occurrence of the corresponding sites in the samples to count the number of occurrences of all sites as the index E-index that reflects the consistency of end distribution in the sample and model. It can be understood that since the DNA end sites only appearing in the samples or non-cancer population must have one of $ai$ or $bi$ equal to $0$, $n$ may also only represent the number of end sites of the cell-free DNA overlapping between the end sites of the cell-free DNA of the samples and the end distribution model of the cell-free DNA of the non-cancer population, such that only $n$ sites overlapped as above are calculated. In addition, the above weighted results may be further normalized. For example, the above results may be normalized by the total number of cell-free DNA in the samples (N$_{\text{sample}}$) and used as E-index, that is, $\text{E} - \text{index} = \frac{1}{N_{sample}} \sum_{i=1}^{n} a_i b_i$. Further, the total number of cell-free DNA in the end distribution model (N$_{\text{model}}$) may be used to perform secondary normalization on the above results and serve as the E-index, that is, $\text{E} - \text{index} = \frac{1}{N_{sample} N_{model}} \sum_{i=1}^{n} a_i b_i$. It can be understood that in this step, in addition to the weighted average model, appropriate statistical methods may also be used to compare the results of the cell-free DNA end site-frequency of the samples in the target genome intervals with the results of the cell-free DNA end site-frequency of the non-cancer population, thus determining the consistency of the two. For example, the correlation of the frequency data of these two groups of specific sites is compared through an appropriate non-parametric test method, such as Pearson correlation, Spearman correlation, and the like, in order to determine the end distribution consistency between the sample and the model.

It should be noted that the end distribution consistency E-index value belongs to a quantitative method of the cfDNA fragmentation model, which may be used alone for cancer diagnosis, or may be used in combination with other biomarkers (including quantitative methods of different cfDNA fragmentation patterns, and markers, such as copy number variation, that are not associated with cfDNA fragmentation patterns).

S230: The classification of the samples is determined based on the comparison results. Specifically, the method for determining the classification of the samples based on the comparison results includes: comparing an E of the samples with a cut off value, where when E-index is greater than or equal to the cut off value, the samples are classified as non-cancer samples; and when the E-index is less than the cut off value, the samples are classified as cancer samples. The cut off value is the boundary value for determining the positive and negative tests to distinguish normal from abnormal. In an embodiment of the present disclosure, different samples are distinguished as the non-cancer control population or the cancer population. The cut off value may be determined by any method well known in the art, where the most commonly used is the receiver operating characteristic curve (ROC curve).

It can be understood that in the sample classification method provided in an embodiment of the present disclosure, the distinction model used is constructed from the non-cancer population. Therefore, a variety of different cancers may be distinguished therefrom. The specific type of cancer may be selected from at least one of brain cancer, head and neck cancer, lung cancer, liver cancer, breast cancer, stomach cancer, colon cancer, rectal cancer, cervical cancer, ovarian cancer, pancreatic cancer, prostate cancer, thyroid cancer, lymphoma, skin cancer, and leukemia. The brain cancer includes primary brain cancer and secondary brain cancer. The primary brain cancer includes glioma and non-glioma (such as meningioma, medulloblastoma, medullary pituitary adenoma, primary CNS lymphoma and central nervous system germ cell tumors, and the like). The secondary brain cancer includes malignant tumors that originate elsewhere but have spread (metastasized) to the brain, such as breast cancer and lung cancer that have metastasized to the brain. The head and neck cancer includes oral cavity/oropharyngeal cancer, nasopharyngeal cancer, nasal cavity/-paranasal sinus cancer, laryngeal cancer/hypopharyngeal cancer, salivary gland cancer, etc. The lung cancer includes small cell lung cancer, non-small cell lung cancer, etc. The liver cancer includes hepatocellular carcinoma, cholangiocarcinoma, hepatoblastoma, etc. The breast cancer includes carcinoma in situ (such as lobular carcinoma in situ, ductal carcinoma in situ), invasive breast cancer (invasive lobular carcinoma, invasive ductal carcinoma), etc. The stomach cancer includes gastric adenocarcinoma, gastrointestinal stromal tumor, gastrointestinal leiomyosarcoma, gastrointestinal carcinoid, gastrointestinal lymphoma, etc. The cervical cancer includes squamous cell carcinoma, adenocarcinoma, etc. The ovarian cancer includes epithelial tumor, blastoma, stromal tumor, etc. The pancreatic cancer includes pancreatic cancer, cystic tumor, acinar cell carcinoma, sarcoma, ampullary cancer, etc. The thyroid cancer includes papillary thyroid carcinoma, follicular thyroid carcinoma, Hearst cell carcinoma, etc. The lymphoma includes Hodgkin lymphoma, non-Hodgkin lymphoma, etc. The skin cancer includes non-melanoma skin cancer and melanoma skin cancer, etc. The leukemia includes acute lymphoblastic leukemia, acute myeloid leukemia, chronic lymphocytic leukemia, chronic myeloid leukemia, etc.

In addition, it should be noted that in some embodiments, the classification method provided in the present disclosure may only exclude the possibility of some samples being "non-cancer", but cannot determine the type of cancer. Cancer diagnosis and tumor traceability belong to two different categories and are applicable to different objects. The pan-cancer classification method provided in an embodiment of the present disclosure is particularly suitable for people who participate in routine physical examinations, to screen out early-stage cancer patients through cfDNA analysis. After completing the classification provided in the present disclosure, it may be necessary to further trace the origin of the tumors to determine the specific cancer type.

To this end, the classification method may also further include the step of confirming the source of the cancer to further classify the samples to complete the traceability of the cancer, or this classification method is combined with other classification methods to further improve the accuracy of cancer diagnosis. Specifically, the analysis may be performed in combination with other data of cell-free DNA or other types of data of subjects. Other data of the cell-free DNA includes, but is not limited to, copy number variation data of the cell-free DNA, methylation data of the cell-free DNA, length distribution data of the cell-free DNA, and end base distribution data of the cell-free DNA, etc. Other types of data of subjects may be marker data, image data, etc., and is analyzed through the model, and the classification of the samples is determined by combining the analysis results and the comparison results. The samples may be classified into the cancer population or the non-cancer population for the subjects to whom the samples belong. It may further be classified into a population suffering from a specific cancer, or a population with cancer metastasis, such as lung cancer liver metastasis, and the like. The markers refer to small molecules (such as amino acids, choline, and the like as metabolic markers), nucleic acids (such as miRNA, mRNA, lncRNA, and the like), proteins, and bacterial groups, etc. associated with one or several specific cancer types. It can be understood that in an embodiment of the present disclosure, the marker data is data of other types of markers that are not associated with the above-mentioned copy number variation, methylation, length distribution, end base distribution, and end distribution of the cell-free DNA, or data of other quantitative methods of the cell-free DNA as markers.

The present disclosure further provides a computer-readable storage medium that stores computer-executable instructions. The computer-executable instructions are used to cause the computer to execute the aforementioned construction method or the aforementioned classification method.

The present disclosure further provides a device that includes a processor and a memory. The memory stores a computer program capable of running on the processor, and the processor implements the aforementioned construction method or the aforementioned classification method when running the computer program.

The memory, as a non-transitory computer-readable storage medium, may be used to store non-transitory software programs and non-transitory computer executable programs, such as the aforementioned construction method or the aforementioned classification method described in an embodiment of the present disclosure. The processor implements the construction of the cancer diagnosis model or the classification of samples by running the non-transient software programs and instructions stored in the memory.

The memory may include a program storage area and a data storage area, where the program storage area may store an operating system and an application program required for at least one function, and the storage data area may store and execute the above programs. In addition, the memory may include a high-speed random access memory and may also include a non-transitory memory, such as at least one disk storage device, flash memory device, or other non-transitory solid-state storage device.

In some embodiments of the present disclosure, the memory optionally includes a memory located remotely relative to the processor, which may be connected to the processor via a network. Examples of the above networks include but are not limited to the Internet, intranets, local area networks, mobile communication networks and combinations thereof.

The non-transitory software programs and instructions required to implement the above evaluation are stored in the memory. When the non-transitory software programs and instructions are executed by one or more processors, the construction of the above cancer diagnosis model or the classification of samples is performed.

The present disclosure further provides a device for constructing a cancer model, including:

an acquisition module configured to obtain sequencing data of cell-free DNA of a non-cancer population;

a comparison module configured to compare the sequencing data of the cell-free DNA of the non-cancer population with reference genomes to obtain corresponding sites of the ends of the cell-free DNA on the reference genomes; and

a model construction module configured to construct the cancer diagnosis model based on the corresponding sites of the ends of the cell-free DNA of the non-cancer population on target genome intervals.

Specifically, constructing the cancer diagnosis model based on the corresponding sites of the ends of the cell-free DNA of the non-cancer population on target genome intervals refers to construct the cancer diagnosis model based on the distribution characteristics of the different sites of the ends of the cell-free DNA of the non-cancer population on the target genome intervals. For example, the end distribution model that represents a site-frequency relationship is constructed by calculating the number of occurrences that different sites of the target genome intervals appear as the ends of the cell-free DNA, and this end distribution model of cfDNA in the non-cancer population is used as a cancer diagnosis model.

The present disclosure further provides a device for diagnosing cancer, including:

an acquisition module configured to obtain end distribution data of cell-free DNA of samples; and

a comparison module configured to compare the end distribution data of the cell-free DNA of the samples with the end distribution data of the cell-free DNA of the non-cancer population and determine whether the samples are cancer samples according to the comparison results.

Specifically, the acquisition module is used to obtain the end distribution data of the cell-free DNA of the samples. The end distribution data refers to the collection of the number of occurrences that different sites of the target genome intervals appear as the ends of the cell-free DNA of the samples. The end distribution data of the cell-free DNA of the non-cancer population refers to the end distribution model constructed based on the number of occurrences that different sites of the target genome intervals appear as the ends of the cell-free DNA of non-cancer population. The comparison module calculates end distribution consistency by the following formula: $E - \text{index} = \sum_{i=1}^{n} a_i b_i$, where $n$ is the number of sites appearing in the end distribution model, $a_i$ is the frequency of site $i$ appearing in the end distribution model, and $b_i$ is the frequency of site $i$ appearing in the ends of the cell-free DNA of the samples. It can be understood that in this comparison module, in addition to using the end distribution consistency E-index described above for comparison, appropriate statistical methods may also be used to compare the results of the cell-free DNA end site-frequency of the samples in the genome intervals with the results of the cell-free DNA end site-frequency of the non-cancer population, thus comparing the consistency of the two.

It can be understood that the device for diagnosing cancer may also include relevant modules for constructing the cancer model, or the corresponding acquisition module and comparison module have a plurality of functions.

Specifically, the device for diagnosing cancer includes:

an acquisition module configured to obtain sequencing data of cell-free DNA of a non-cancer population and end distribution data of the cell-free DNA of the samples;

a comparison module configured to compare the sequencing data of the cell-free DNA of the non-cancer population with the reference genomes to obtain corresponding sites of the ends of the cell-free DNA on the reference genomes, and configured to compare the end distribution data of the cell-free DNA of the samples with the cancer diagnosis model, and determine whether the samples are cancer samples according to the comparison results; and

a model construction module configured to construct the cancer diagnosis model based on the corresponding sites of the ends of the cell-free DNA of the non-cancer population on target genome intervals.

The device implementation described above is only illustrative, and the modules described as separate components may or may not be physically separated, that is, they may be located in one place, or they may be distributed to multiple network units. Some or all of the modules may be selected according to actual needs to achieve the objectives of the solution of this embodiment.

It can be understood that all or some of the steps disclosed above may be implemented as software, firmware, hardware, and appropriate combinations thereof. Some or all of the physical components may be implemented as software executed by a processor, such as a central processing unit, a digital signal processor, or a microprocessor, or implemented as hardware, or as an integrated circuit, such as an application specific integrated circuit. Such software may be distributed on computer-readable media, which may include computer storage media (or non-transitory media) and communication media (or transitory media). It can be understood that the computer storage media include volatile and nonvolatile, removable and non-removable media implemented in any method or technology for storage information, such as computer readable instructions, data structures, program modules or other data. The computer storage media include, but are not limited to, RAM, ROM, EEPROM, flash memory or other memory technology, CD-ROM, digital versatile disk (DVD) or other optical disk storage, magnetic cassettes, tapes, disk storage or other magnetic storage devices, or any other media that may be used to store the desired information and that may be accessed by a computer.

Furthermore, it can be understood that the communication media typically include computer readable instructions, data structures, program modules or other data in a modulated data signal such as a carrier wave or other transport mechanisms, and may include any information delivery media.

The sequencing method of the present disclosure will be described below with reference to specific examples.

Example 1

I. Construction of cancer diagnosis model

1. Sample source

Twenty-four volunteers were enrolled from the hospital's physical examination population. The physical examination results confirmed that there were no cancer patients. All volunteers signed informed consent forms and underwent ethical review before sample collection.

2. Collection and processing of peripheral blood

A blood collection tube containing anticoagulant (EDTA) was used to collect peripheral blood (6 mL) from veins. A centrifuge was used to centrifuge at 4°C and 1,600 × g for 15 minutes. An upper liquid was aspirated and transferred to a new centrifuge tube. The centrifuge was used again to centrifuge at 4°C and 16,000 × g for 15 minutes, and then the upper liquid (i.e., plasma) was aspirated and transferred to a cryovial, and stored in a -80°C refrigerator.

3. Extraction and sequencing of cfDNA

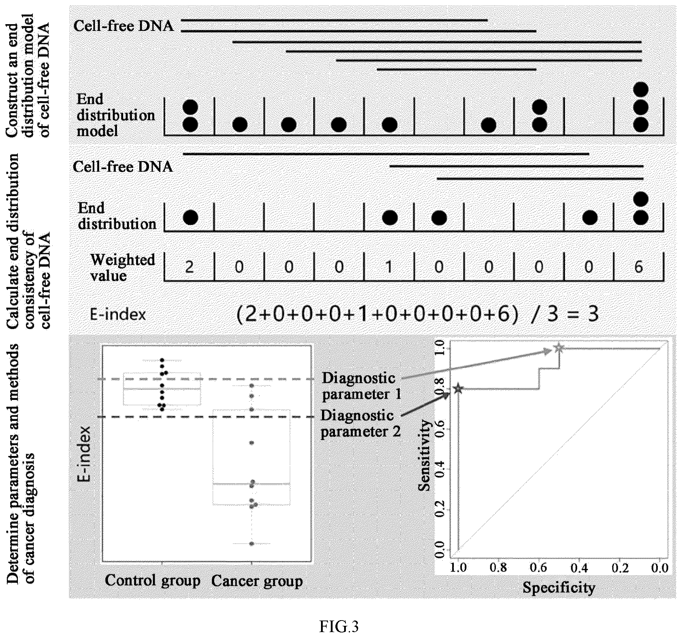

Commercial kits and devices were used to extract cfDNA from plasma, construct sequencing libraries, and perform high-throughput sequencing (paired-end). The raw sequencing data removed sequencing adapters, low-quality loops, and repetitive sequences, and then was compared to the whole human genome to remove repetitive sequences and low-quality comparison results to obtain the final comparison results. The positions of both ends of each cfDNA on the genome were extracted, namely, the end site information of cfDNA. Referring to FIG. 3, the number of that each site of the reference genome was correspondingly positioned to the cfDNA end of this site was counted, and a statistical table of the number that each site in the genome served as the cfDNA end was obtained to construct a site-frequency end distribution model.

II. Sample classification

1. Calculation of cfDNA end distribution consistency (E-index)

For each sample to be tested, the cfDNA end information was extracted according to the method for constructing a cancer diagnosis model, and the total number ($N_{sample}$) of cfDNA fragments in the comparison results was recorded.

For all sites in the genomes, the number serving as the cfDNA end in the sample to be tested was weighted with the number serving as the cfDNA end in the end distribution model as the weight, then the $N_{sample}$ was used to normalize the weighted value, which was used as the E-index to represent the consistency of the cfDNA end distribution of the samples to be tested and that of the non-cancer population. Referring to the following formula:

$$\text{E} - \text{index} = \frac{1}{N_{sample}} \sum_{i=1}^{n} a_i b_i$$

where $n$ is the number of sites appearing in the end distribution model, $a_i$ is the frequency of site $i$ appearing in the end distribution model, and $b_i$ is the frequency of site $i$ appearing in the ends of the cell-free DNA of the samples; $N_{sample}$ is the total number of cfDNA in the samples.

The calculation method of E-index was further illustrated with reference to FIG. 3. It was assumed that there were 10 sites in the model. The number of occurrences that these 10 sites appeared as cfDNA ends of the non-cancer population was 2, 1, 1, 1, 1, 0, 1, 2, 0, 3 successively. At the same time, it was assumed that the number of occurrences that these 10 sites appeared as cfDNA ends was 1, 0, 0, 0, 1, 1, 0, 0, 1, 2 successively. The frequency in the model was used as a weight, multiplied by the frequency in the samples and normalized by the number of cfDNA to be tested (3) in the samples. The calculation process is as follows:

$$\text{E-index} = (2 \times 1 + 1 \times 0 + 1 \times 0 + 1 \times 0 + 1 \times 1 + 0 \times 1 + 1 \times 0 + 2 \times 0 + 0 \times 1 + 3 \times 2)/3 = 3.$$

## 2. Determination of cutoff value

Referring to FIG. 3, non-cancer patients (not coinciding with the patients enrolled when constructing the model) were enrolled as the control group and cancer patients (may be a single cancer type or multiple cancer types) were enrolled as the cancer group to calculate the E-index value of cfDNA of each patient sample. Due to the presence of tumor-derived cfDNA in cancer patients, on the whole, the E-index value of the cancer patients should be lower than that of non-cancer patients, and thus a cutoff value might be used to differentiate and achieve cancer diagnosis.

The receiver operating characteristic curve (ROC) was used to evaluate the accuracy, sensitivity, specificity and other indicators when using different cutoff values as diagnostic parameters for cancer diagnosis, and the cutoff values were determined according to actual needs, for example, the test may require specificity >99% for community screens, and may require sensitivity >95% for high-risk population to determine the cutoff value needed to meet specific needs.

The AUC (Area Under the Curve) value was used to evaluate the overall performance of the classification: the higher the AUC, the better the effect.

## 3. Classification of samples

After determining the cutoff value, the E-index value of its cfDNA was calculated. If the E-index value was lower than the cutoff value, the person was considered a cancer patient, otherwise the person was considered a non-cancer patient.

Data sets from different sources were used to classify samples, and the results were as follows:

(1) Data set 1 contained sample data of 10 non-cancer patients, 10 liver cancer patients, and 10 lung cancer patients (for data set source, refer to Liang et al. Whole-genome sequencing of cell-free DNA yields genome-wide read distribution patterns to track tissue of origin in cancer patients. Clin Transl Med. 2020 Oct; 10(6):e177).

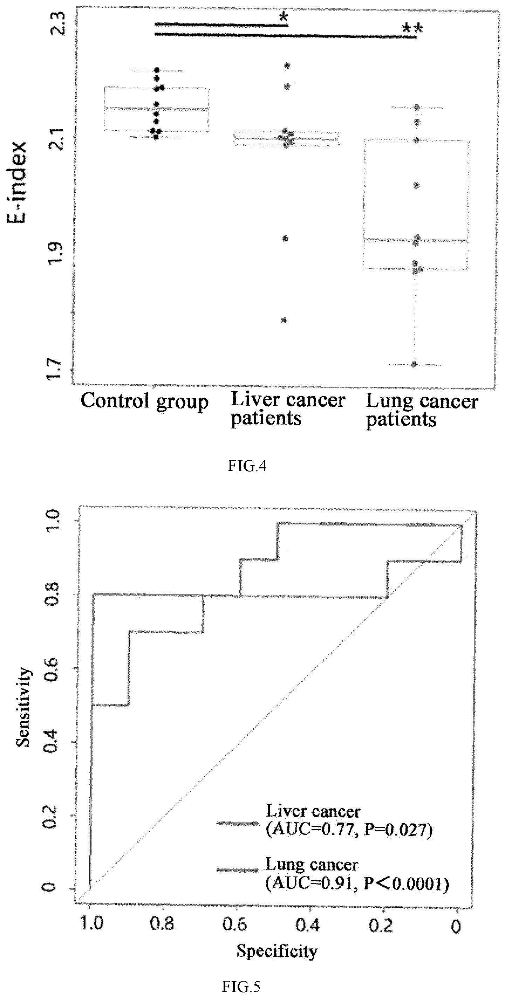

The E-index values of these patients were calculated according to the consistency calculation method provided above, as shown in FIG. 4. It could be seen from FIG. 4 that compared with non-cancer patients as the control group, the E-index values of liver cancer patients were significantly lower overall (P<0.05), and the same was true for lung cancer patients (P<0.01).

Refer to FIG. 5 for the ROC analysis results. The upper part was the ROC curve of lung cancer, and the lower part was the ROC curve of liver cancer. E-index was used for cancer diagnosis, the AUC value in liver cancer patients reached 0.77, while in lung cancer patients, the AUC value reached 0.91, indicating that E-index may be used to effectively distinguish cancer patients from non-cancer patients. When the specificity requirement was 0.90, the sensitivity of cancer diagnosis was 0.70 (liver cancer) and 0.80 (liver cancer).

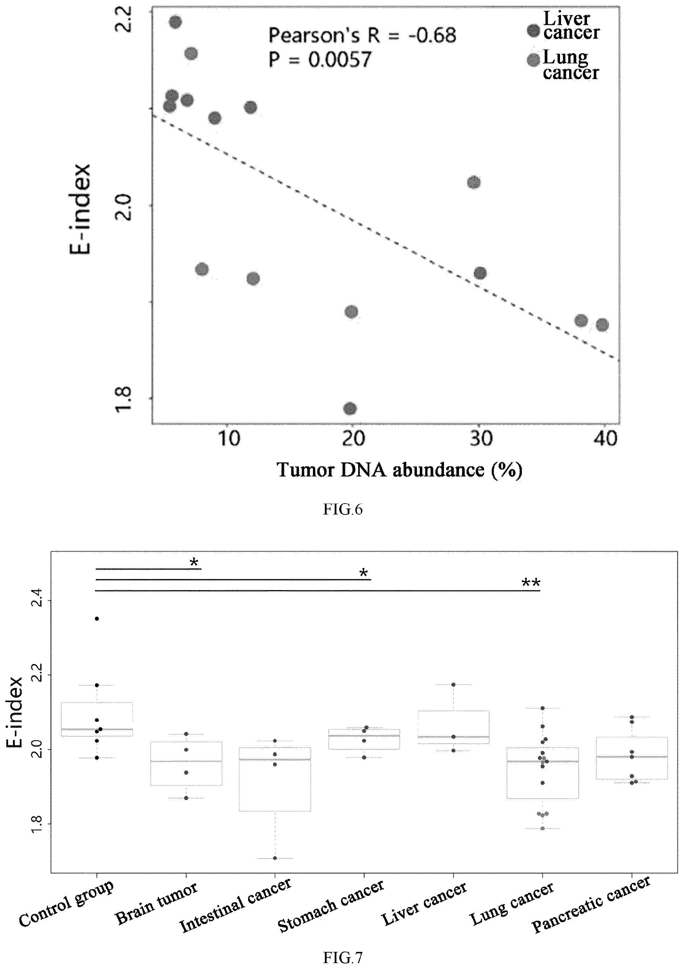

The ichorCNA algorithm was used to detect cfDNA somatic copy number variation (SCNA) in the samples of the above 20 patients and tumor DNA abundance (TFx) was quantitatively calculated to detect the correlation between tumor DNA abundance and consistency E-index. The results were shown in FIG. 6. It could be seen from FIG. 6 that in cancer patients, the E-index value was negatively correlated with the proportion of tumor-derived cfDNA. Therefore, the size, malignancy and other information of the tumors for patients may be evaluated through E-index.

(2) Data set 2 contained sample data of 7 non-cancer patients (including some hepatitis B carriers), 4 brain tumor patients, 4 intestinal cancer patients, 4 stomach cancer patients, 3 liver cancer patients, and 15 lung cancer patients, and 7 pancreatic cancer patients (for data set source, refer to Song et al. 5-Hydroxymethylcytosine signatures in cell-free DNA provide information about tumor types and stages. Cell Res. 2017 Oct; 27(10):1231-1242).

After calculating the E-index values of these people according to the consistency calculation method provided above, since the sample size of some cancer patients was small, they were first combined to compare the cancer group (brain tumor + intestinal cancer + stomach cancer + liver cancer + lung cancer + pancreatic cancer) with the control group (non-cancer population). There was a significant difference (Mann-Whitney Rank-sum test, P=0.0064) in the overall E-index value between the two groups.

Subsequently, they were grouped according to cancer type to compare the difference between the E-index of different cancer types and the control group. The results were shown in FIG. 7. It could be seen that patients with most cancer types had the overall E-index lower than the non-cancer population.

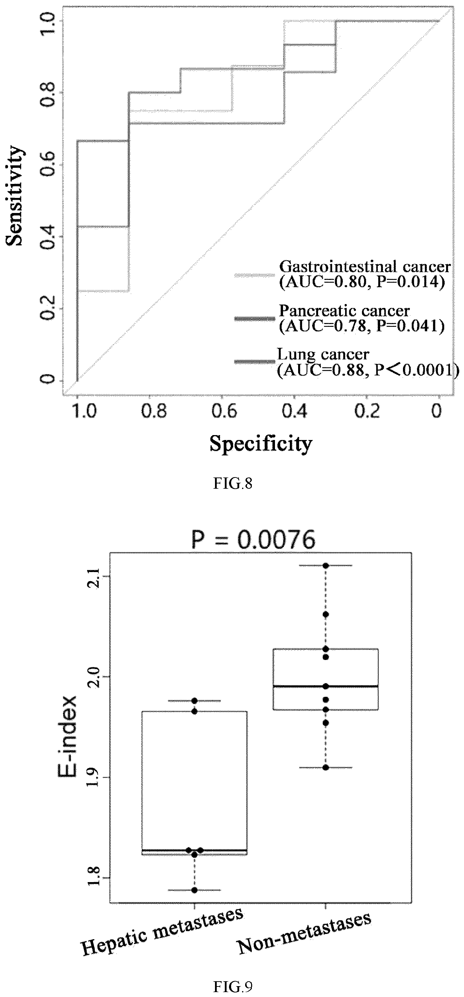

Several cancer types with more patients were selected, including gastrointestinal cancer patients (including colorectal cancer and stomach cancer patients), pancreatic cancer patients, and lung cancer patients, and ROC analysis was conducted separately. The results were shown in FIG. 8. In the specificity ranging from 0.8 to 0.6, from top to bottom were the ROC curves of lung cancer, gastrointestinal cancer and pancreatic cancer. It could be seen from FIG. 8 that E-index was used for cancer diagnosis, and the AUCs were respectively 0.80 (gastrointestinal cancer), 0.78 (pancreatic cancer), and 0.88 (lung cancer), indicating that E-index may be used to effectively distinguish cancer patients from non-cancer population. When the specificity requirement was 0.85, the sensitivity was 0.75 (gastrointestinal cancer), 0.71 (pancreatic cancer) and 0.80 (lung cancer), respectively.

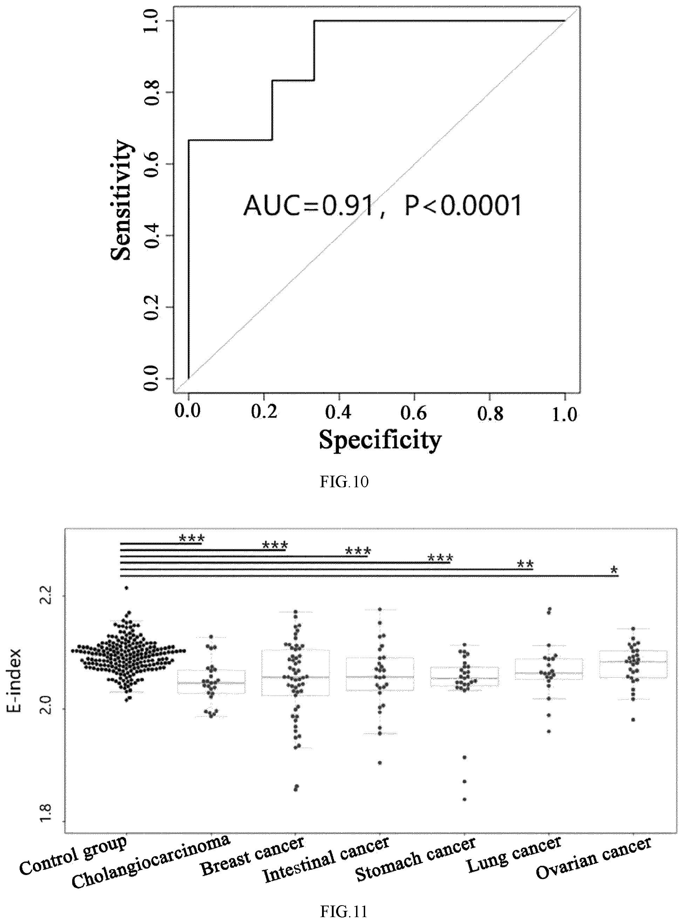

In addition, in the 15 lung cancer patients, some have already developed tumor metastasis (liver metastasis). Referring to FIG. 9, the E-index values of these patients were also significantly lower than those of lung cancer patients without metastasis. Referring to FIG. 10 at the same time, the AUC value of E-index for patients with metastasis and non-metastasis was 0.91. These results indicate that E-index may be used to predict whether tumors will metastasize, especially for predicting lung cancer metastasis, particularly for predicting liver metastasis of lung cancer.

(3) Data set 3 contained sample data of 231 non-cancer persons, 26 cholangiocarcinoma patients, 54 breast patients, 27 colorectal cancer patients, 27 stomach cancer patients, 79 lung cancer patients, and 28 ovarian cancer patients (for data set source, refer to Cristiano et al. Genome-wide cell-free DNA fragmentation in patients with cancer. Nature 2019; 570:(385-389)).

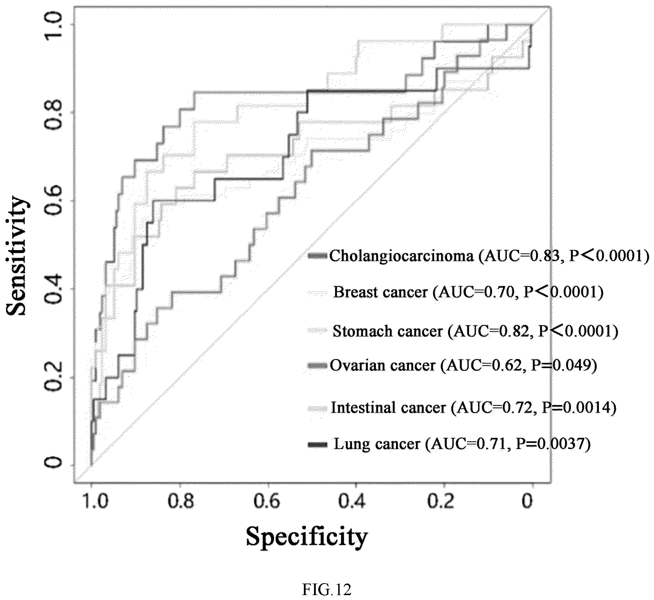

The E-index values of these patients were calculated according to the consistency calculation method provided above, and the differences in E-index values of different types were compared. The results were shown in FIG. 11. It could be seen from FIG. 11 that these cancer patients had the overall E-index lower than the non-cancer patients. The ROC analysis results were shown in FIG. 12. When the specificity was 0.6, from top to bottom were the ROC curves of cholangiocarcinoma, stomach cancer, intestinal cancer, breast cancer, lung cancer, and ovarian cancer. It could be seen from FIG. 12 that E-index was used for cancer diagnosis, and the AUC value was between 0.62 and 0.83, indicating that E-index may be used to effectively distinguish cancer patients from non-cancer population. When the specificity requirement was 0.90, the sensitivity was between 0.15 and 0.7.

Example 2

This example provides a device for diagnosing cancer, including:

an acquisition module configured to obtain a site-frequency statistical table composed of the number of occurrences that the cell-free DNA of the samples appear the ends of the cell-free DNA at each site in the whole human genome as its end distribution data; and

a comparison module configured to compare the site-frequency of the samples with the site-frequency of the diagnosis model, test the consistency between the two above through the Pearson correlation coefficient, and determine whether the subject has a cancer according to the cut off value of R in the model.

The site-frequency of the diagnosis model was constructed by comparing the sequencing results of cell-free DNA samples from 1,000 non-cancer population.

The device for diagnosing cancer may achieve similar effects of diagnosing cancer in different data sets as in Example 1, which will not be described again here.

Example 3

This example provides a device for diagnosing cancer, including:

an acquisition module configured to obtain a site-frequency statistical table composed of the number of occurrences that the cell-free DNA of the samples appear the ends of the cell-free DNA at each site in the whole human genome as its end distribution data; and obtain the copy number of a plurality of genes of the cell-free DNA of the samples as copy number variation data; and

a comparison module configured to compare the site-frequency of the samples with the site-frequency of the diagnosis model, calculate the E-index value, calculate the CNV risk score based on the copy number variation data, and combine the E-index value and the CNV risk score to determine whether the subject has a cancer. The selection of genes and the calculation method of CNV risk score may be learned from the literature and will not be described in detail here.

Based on the above embodiments, it can be seen that the cancer diagnosis model and corresponding classification method provided in the present disclosure may be used for multiple cancer types, with high universality and wide application. At the same time, the diagnostic accuracy of using the above method is high, especially in patients with early-stage cancer, which is greatly improved compared with traditional methods. In addition, the data used to implement the above solutions may only be obtained by commonly used whole genome sequencing. There are no special requirements for experimental protocols and sequencing platforms, with low operational complexity and low cost, making it suitable for clinical promotion.

The present disclosure has been described in detail above with reference to the embodiments. However, the present disclosure is not limited to the above-mentioned embodiments. Various changes can be made within the knowledge scope of those of ordinary skill in the art without departing from the spirit of the present disclosure. In addition, embodiments of the present disclosure and features in the embodiments may be combined with each other without conflicts.

**Claims**

1. A construction method for a cancer diagnosis model, comprising the steps of:

   obtaining sequencing data of cell-free DNA of a non-cancer population;
   comparing the sequencing data of the cell-free DNA of the non-cancer population with reference genomes to obtain corresponding sites of the ends of the cell-free DNA on the reference genomes; and
   constructing the cancer diagnosis model based on the corresponding sites of the ends of the cell-free DNA on target genome intervals.

2. The construction method according to claim 1, wherein the ends of the cell-free DNA comprise at least one of upstream ends and downstream ends.

3. The construction method for according to claim 1, wherein the target genome interval is a whole human genome or a set interval of the whole human genome.

4. The construction method according to claim 1, wherein the method for constructing the cancer diagnosis model based on the corresponding sites of the ends of the cell-free DNA on the target genome intervals comprises:
   calculating the number of occurrences that different sites of the target genome intervals appear as of the ends of the cell-free DNA to construct an end distribution model that represents a site-frequency relationship, namely being the cancer diagnosis model.

5. A sample classification method, comprising the steps of:

   obtaining end distribution data of cell-free DNA of samples;
   comparing the end distribution data of the cell-free DNA of the samples with the end distribution data of the cell-free DNA of a non-cancer population; and
   determining the classification of the samples based on comparison results.

6. The classification method according to claim 5, wherein the method for obtaining the end distribution data of the cell-free DNA of the samples comprises:

   obtaining sequencing data of the cell-free DNA of the samples;
   comparing the sequencing data of the cell-free DNA of the samples with reference genomes to obtain corresponding sites of the ends of the cell-free DNA of the samples on the reference genomes; and
   calculating the number of occurrences that different sites of target genome intervals appear as the ends of the cell-free DNA of the samples.

7. The classification method according to claim 6, wherein the end distribution data of the cell-free DNA of the non-cancer population is obtained by:

   obtaining sequencing data of the cell-free DNA of the non-cancer population;
   comparing the sequencing data of the cell-free DNA of the non-cancer population with the reference genomes to obtain the corresponding sites of the ends of the cell-free DNA of the non-cancer population on the reference genomes; and

calculating the number of occurrences that different sites of the target genome intervals appear as the ends of the cell-free DNA of the non-cancer population to construct an end distribution model that represents a site-frequency relationship.

8. The classification method according to claim 7, wherein the method of comparison comprises comparing a consistency of the number of occurrences that different sites of the target genome intervals appear as the ends of the cell-free DNA of the samples and the number of occurrences that different sites of the target genome intervals appear as the ends of the cell-free DNA of the non-cancer population;

the consistency is compared by comparing the correlation of both the number of occurrences that different sites of the target genome intervals appear as the ends of the cell-free DNA of the samples and the number of occurrences that different sites of the target genome intervals appear as the ends of the cell-free DNA of the non-cancer population or calculating an end distribution consistency by the following formula:

$$\mathrm{E} - \mathrm{index} = \sum_{i=1}^{n} a_i b_i$$

and

wherein $n$ is the number of sites appearing in the end distribution model, $a_i$ is the frequency of site $i$ appearing in the end distribution model, and $bi$ is the frequency of site $i$ appearing in the ends of the cell-free DNA of the samples.

9. The classification method according to claim 8, wherein the method for determining the classification of the samples based on the comparison results comprises:
   comparing the end distribution consistency of the samples with a cut off value, wherein when the end distribution consistency is greater than or equal to the cut off value, the samples are classified as non-cancer samples; and when the end distribution consistency is less than the cut off value, the samples are classified as cancer samples.

10. The classification method according to claim 9, wherein the cancer is selected from at least one of brain cancer, head and neck cancer, lung cancer, liver cancer, breast cancer, stomach cancer, colon cancer, rectal cancer, cervical cancer, ovarian cancer, pancreatic cancer, prostate cancer, thyroid cancer, lymphoma, skin cancer, and leukemia.

11. The classification method according to any one of claims 5 to 10, further comprising obtaining at least one of copy number variation data of cell-free DNA, methylation data of cell-free DNA, length distribution data of cell-free DNA, and end base distribution data of cell-free DNA, marker data, and image data of the samples for analysis, and determining the classification of the samples by combining analysis results with the comparison results.

12. A cancer diagnosis model, being constructed using the construction method described in any one of claims 1 to 4.

13. A computer-readable storage medium, storing computer-executable instructions, wherein the computer-executable instructions are used to cause the computer to execute the construction method according to any one of claims 1 to 4 or the classification method according to any one of claims 5 to 11.

14. A device, comprising a processor and a memory, wherein the memory stores a computer program capable of running on the processor, and the processor implements the construction method according to any one of claims 1 to 4 or the classification method according to any one of claims 5 to 11 when running the computer program.

15. A device for constructing a cancer diagnosis model, comprising:

an acquisition module configured to obtain sequencing data of cell-free DNA of a non-cancer population;
a comparison module configured to compare the sequencing data of the cell-free DNA of the non-cancer population with reference genomes to obtain corresponding sites of the ends of the cell-free DNA on the reference genomes; and
a model construction module configured to construct the cancer diagnosis model based on the corresponding sites of the ends of the cell-free DNA on target genome intervals.

16. A device for diagnosing cancer, comprising:

an acquisition module configured to obtain end distribution data of cell-free DNA of samples; and
a comparison module configured to compare the end distribution data of the cell-free DNA of the samples with the end distribution data of the cell-free DNA of the non-cancer population and determine whether the samples are cancer samples according to the comparison results.

obtaining sequencing data of cell-free DNA of a non-cancer population

S110

comparing the sequencing data of the cell-free DNA with reference genomes to obtain corresponding sites of the ends of the cell-free DNA on the reference genomes

S120

constructing a cancer diagnosis model based on the corresponding sites of the ends of the cell-free DNA on target genome intervals

S130

FIG.1

obtaining end distribution data of cell-free DNA of samples

S210

comparing the end distribution data of the cell-free DNA of the samples with the end distribution data of the cell-free DNA of the non-cancer population

S220

determining the classification of the samples based on comparison results

S230

FIG.2

FIG.3

FIG.4

FIG.5

FIG.6

FIG.7

FIG.8

FIG.9

FIG.10

FIG.11

FIG.12

## INTERNATIONAL SEARCH REPORT

| International application No. |
|---|
| **PCT/CN2022/116248** |

| A. | CLASSIFICATION OF SUBJECT MATTER |
|---|---|

G16H 50/20(2018.01)i

According to International Patent Classification (IPC) or to both national classification and IPC

| B. | FIELDS SEARCHED |
|---|---|

Minimum documentation searched (classification system followed by classification symbols)

G16H; G16B

Documentation searched other than minimum documentation to the extent that such documents are included in the fields searched

Electronic data base consulted during the international search (name of data base and, where practicable, search terms used)

CNTXT; ENTXT; ENTXTC; DWPI; CNKI: 次数, 频次, 比较, 位点, 位置, 诊断, 模型, 末端, 基因组, 游离DNA, 分布数据, 分布模型, 分类, number, frequency, compare, site, location, diagnostic, model, end, genome, episomal DNA, distribution data, distribution model, classification

| C. | DOCUMENTS CONSIDERED TO BE RELEVANT |
|---|---|

| Category* | Citation of document, with indication, where appropriate, of the relevant passages | Relevant to claim No. |
|---|---|---|
| X | CN 110272985 A (GUANGZHOU XIONGJI BIOLOGICAL INFORMATION TECHNOLOGY CO., LTD.) 24 September 2019 (2019-09-24) claims 6-9, and description, paragraphs 41-89 | 5-11, 16 |
| Y | CN 113981081 A (SUN YAT-SEN UNIVERSITY) 28 January 2022 (2022-01-28) claims 5-10, and description, paragraphs 33-54 | 1-4, 12-15 |
| Y | CN 110272985 A (GUANGZHOU XIONGJI BIOLOGICAL INFORMATION TECHNOLOGY CO., LTD.) 24 September 2019 (2019-09-24) claims 6-9, and description, paragraphs 41-89 | 1-4, 12-15 |

☐ Further documents are listed in the continuation of Box C.    ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **13 January 2023** | **20 January 2023** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2022/116248**

| Patent document cited in search report | | | Publication date (day/month/year) | Patent family member(s) | Publication date (day/month/year) |
|---|---|---|---|---|---|
| CN | 110272985 | A | 24 September 2019 | None | |
| CN | 113981081 | A | 28 January 2022 | None | |

Form PCT/ISA/210 (patent family annex) (January 2015)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LIANG et al.** Whole-genome sequencing of cell-free DNA yields genome-wide read distribution patterns to track tissue of origin in cancer patients. *Clin Transl Med.*, October 2020, vol. 10 (6), e177

- **SONG et al.** 5-Hydroxymethylcytosine signatures in cell-free DNA provide information about tumor types and stages. *Cell Res.*, October 2017, vol. 27 (10), 1231-1242
- **CRISTIANO et al.** Genome-wide cell-free DNA fragmentation in patients with cancer. *Nature*, 2019, vol. 570 (385-389)